# EUROPÄISCHE PATENTSCHRIFT

(11) **EP 1 169 067 B1**
(45) Veröffentlichungstag und Bekanntmachung des Hinweises auf die Patenterteilung: **04.06.2003**
(21) Anmeldenummer: 00915188.7
(22) Anmeldetag: 24.03.2000
(51) Int. Cl.: A61L 2/07, B67B 1/03

(54) **REINIGUNGS- UND STERILISIERMASCHINE**
CLEANING AND STERILIZING MACHINE
MACHINE DE NETTOYAGE ET DE STERILISATION

(30) Priorität: 13.04.1999 DE 19916720
(43) Veröffentlichungstag der Anmeldung: 09.01.2002
(73) Patentinhaber: SMEJA GMBH & CO. KG, D-47638 Straelen (DE)
(72) Erfinder: WIECZOREK, Joachim, D-47877 Willich-Anrath (DE)
(74) Vertreter: Knauf, Rudolf, Dipl.-Ing.
(86) Internationale Anmeldenummer: EP0002615
(87) Internationale Veröffentlichungsnummer: WO00061199

(56) Entgegenhaltungen:
- EP-A- 0 544 161
- EP-B- 0 418 450
- DE-A- 4 409 659
- DE-A- 19 824 724
- GB-A- 2 212 387

## Beschreibung

Die Erfindung betrifft eine Reinigungs- und Sterilisiermaschine für kleine Gegenstände wie Verschlußelemente von pharmazeutischen Gefäßen, die aus einem von einer horizontalen Welle in einem Träger getragenen und um die Achse der Welle schwenkbar gelagerten Behandlungsbehälter, der einen trichterförmigen Oberteil mit einer darin angeordneten, durch ein Ventil verschließbaren Be- und Entladeöffnung aufweist und an dem von der Welle getragene Zu- und Abfuhrleitungen für mindestens ein Behandlungsmedium wie Wasser, Dampf, Druckluft angeschlossen sind, besteht.

Bei der Herstellung pharmazeutischer Produkte werden extrem hohe Anforderungen hinsichtlich der Reinheit von Teilen von Verpackungen, z.B. Gummistopfen, gestellt. Ein Waschen der Gegenstände, z.B. in einer Trommelwaschmaschine, und ein anschließendes Trocknen und Sterilisieren der Gegenstände, genügt den Anforderungen bei weitem nicht. Um den Anforderungen mehr zu genügen, hat man spezielle Wasch-, Sterilisier- und Trockenapparate entwickelt. Wegen der Kontaminationsgefahr kommt dabei den Be- und Entladevorrichtungen, durch die die zu reinigenden Gegenstände in die Maschine gefüllt bzw. die gereinigten Gegenstände aus der Maschine entnommen werden, eine besondere Bedeutung zu.

Aus der EP 0 418 450 ist eine Reinigungs- und Sterilisiermaschine der eingangs genannten Art bekannt, in der der Behandlungbehälter über die Be- und Entladeöffnung mit einem die zu behandelnden Gegenstände enthaltenen Container lösbar, dicht und tragfähig verbunden wird. Die zu behandelnden Gegenstände fallen nach Verschwenken des Behandlungsbehälters um 180° aus dem Container in den nun unter dem Container befindlichen Behandlungsbehälter, wo sie gemeinsam mit dem Innenraum des angeschlossenen Containers gereinigt und steril gemacht werden. Durch erneutes Verschwenken werden die gereinigten Gegenstände wieder zurück in den Container befördert und dieser nach hermetischen Verschließen vom Behandlungsbehälter abgekuppelt. Zwar besitzt diese Reinigungs- und Sterilisiermaschine den Vorteil, daß sie nicht in einer sterilen Kammer untergebracht sein muß, nachteilig ist jedoch die große Bauhöhe, die erforderlich ist, um ein Verschwenken der mit dem Container verbunden Behandlungsbehälters zu ermöglichen. Als-nachteilig wird ferner der das dem Reinigungs- und Sterilisiervorgang nachgeschaltete Umfüllen der sterilen Gegenstände aus dem Behandlungsgefäß in den Container empfunden.

Aus einem nicht druckschriftlich veröffentlichtem Stand der Technik ist ferner eine Reinigungs- und Sterilisiermaschine mit vergleichsweise niedriger Bauhöhe bekannt, bei der der Behandlungsbehälter an einer an einem Maschinengehäuse befestigten Tragevorrichtung lösbar aber nicht drehbar befestigt ist. Der Behandlungsbehälter weist im oberen Bereich eine durch ein Klappenventil verschließbare Beladeöffnung für die zu behandelnden Gegenstände und im unteren Bereich eine durch ein Klappenventil verschließbare Entladeöffnung für die zu behandelnden Gegenstände auf. Zu- und Ableitungen für die Behandlungsmedien Wasser, Dampf etc. sind stationär an dem Maschinengehäuse befestigt und werden über Kupplungen mit dem Behandlungsbehälter verbunden. In dem Behandlungsbehälter ist ein als Sieb ausgebildeter Korb drehbar angeordnet, der zur Aufnahme der zu reinigenden Gegenstände dient. Der Korb hat ein trichterförmiges Teil, so daß er durch Verdrehen in Korrespondenz mit der Be- oder Entladeöffnung gebracht werden kann, um den Behandlungsbehälter mit den zu behandelnden Gegenständen zu beladen bzw. ihn zu entladen. Nach erfolgter Behandlung wird-der nach außen hermetisch verschlossene Behandlungsbehälter von der Tragevorrichtung des Maschinengehäuses entfernt und kann als solcher zur Verbrauchsstelle der Gegenstände, etwa zu einer Arzneimittelabfüllanlage, transportiert werden. Nachteilig an der bekannten Reinigungs- und Sterilisiermaschine ist jedoch ihre aufwendige Konstruktion mit dem im Inneren des Behandlungsbehälters angeordneten drehbaren Korb und zwei separaten Be- und Entladeöffnungen.

Aufgabe der Erfindung ist es, eine Reinigungs- und Sterilisiermaschine zu schaffen, die eine vergleichsweise geringe Bauhöhe erfordert und sich durch eine einfache und kostengünstige Konstruktion auszeichnet.

Diese Aufgabe wird bei einer Reinigungs- und Sterilisiermaschine der eingangs genannten Art dadurch gelöst, daß die Welle eine Tragevorrichtung aufweist, in der der Behandlungsbehälter lösbar gehalten ist, und die Zu- und Abfuhrleitungen über Kupplungen lösbar mit an dem Behandlungsbehälter angeordneten Ventilen verbunden sind.

Dadurch, daß der mit den zu reinigenden Gegenständen befüllte Behandlungsbehälter nach erfolgter Behandlung nach außen hermetisch abgeschlossen und von den übrigen Bestandteilen der Reinigungs- und Sterilisiermaschine abgetrennt werden kann, entfallen die aufwendigen und mit einer möglichen Kontamination der zu reinigenden Gegenstände und der Maschine verbundenen Be- und Entladevorgänge an der Reinigungs- und Sterilisiermaschine. Die durch die Be- und Entladevorgänge bedingte große Bauhöhe der bekannten Reinigungs- und Sterilisiermaschinen wird dadurch vermieden. Der Behandlungsbehälter kann an einem beliebigen Ort in nicht steriler Umgebung mit den zu reinigenden Gegenständen befüllt und nach der Reinigung in hermetisch verschlossenen sterilen Zustand direkt an die Verbrauchsstelle der Gegenstände transportiert werden. Der Behandlungsbehälter zeichnet sich durch eine einfache Konstruktion aus. Wegen des Fehlens beweglicher Teile ist er verschleißarm. Die Beladeöffnung wird gleichzeitig als Entladeöffnung verwendet. Der aus dem Stand der Technik bekannte, im Inneren des Behandlungsbehälter drehbar angeordnete Korb ist nicht erforderlich.

Im folgenden wird die Erfindung anhand einer ein Ausführungsbeispiel darstellenden Zeichnung in schematischer Darstellung erläutert. Im einzelnen zeigen
- Fig. 1: die Reinigungs- und Sterilisiermaschine in Seitenansicht,
- Fig. 2: einen Teil der in Fig. 1 abgebildeten Maschine mit einem um 180° geschwenkten Behandlungsbehälter nach dein Reinigungs- und Sterilisiervorgang in Seitenansicht,
- Fig. 3: den von der Tragevorrichtung und den Zu- und Abfuhrleitungen der Reinigungs- und Sterilisiermaschine abgekuppelten, auf einem Fahrzeug mit Tragegestell befestigten Behandlungsbehälter der Fig. 2 in Seitenansicht.

Die in nicht sterilisierter Umgebung aufgestellte Reinigungs- und Sterilisiermaschine umfaßt einen Behandlungsbehälter 1 und eine in einem Maschinengehäuse 2 schwenkbar gelagerte horizontale Welle 3, auf der der Behandlungsbehälter 1 gelagert ist. Die horizontale Welle 3 tragt eine Gabel 4. Seitlich am Behandlungsbehälter 1 sind rohrformige Schienen 5 ausgebildet, in die die Äste der an der Welle 3 befestigten Gabel 4 arretierbar einschiebbar sind.

Der Behandlungsbehälter 1 hat ein trichterförmiges Oberteil 6 mit einer durch ein verschwenkbares Klappenventil 7 verschlossenen Be- und Entladeöffnung 8. An der Unterseite des Behandlungsbehälters 1 ist eine eine durch ein Ventil 9 verschließbare Öffnung 10 vorgesehen.

Die Be- und Entladeöffnung 8 an der Oberseite des Behandlungsbehälters 1 und die Öffnung 10 an der Unterseite des Behandlungsbehälters 1 sind jeweils auf der dem Behälter abgewandten Seite eine Kupplung 11,12 vorgesehen. An den entsprechenden Kupplungsteilen des Behandlungsbehälters 1 sind Zu- und Ableitungen 13,14 für die Behandlungsmedien Wasser, Dampf und Druckluft dicht und selbsttragend anschließbar, die von den Medienanschlüssen im Maschinengehäuse 2 kommen.

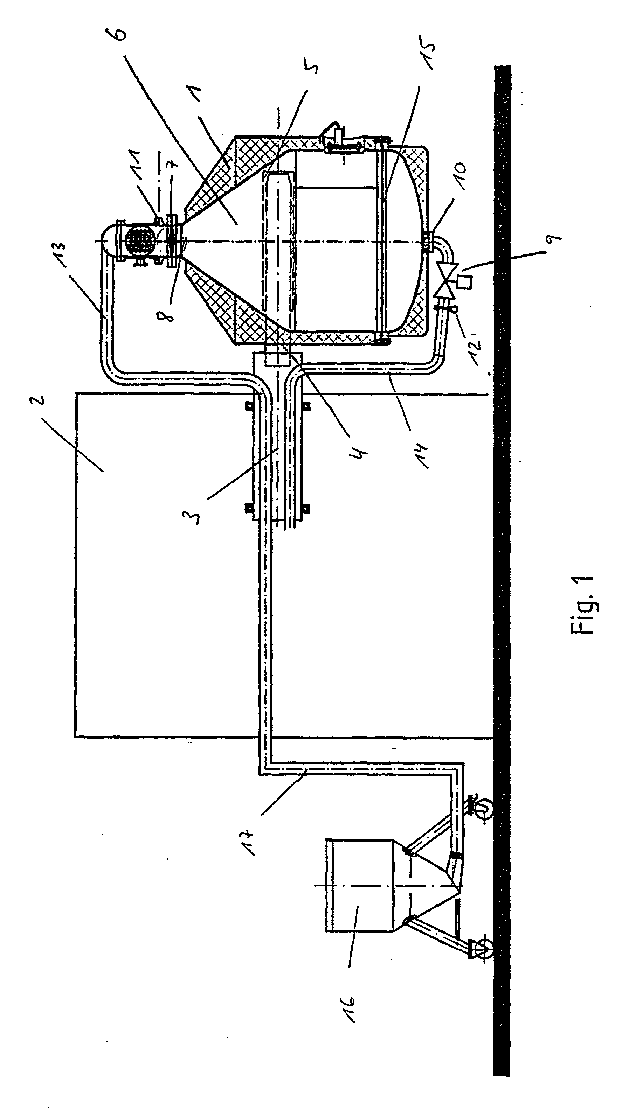

In dem in Behandlungsposition gemäß Fig. 1 befindlichen Behandlungsbehälter 1 liegen die zu behandelnden Gegenstände auf einem horizontal zwischen den beiden Öffnungen 8,10 stationär angeordneten Feinstsieb (Pulsator) 15, das sich über den gesamten Querschnitt des Behandlungsbehälters 1 erstreckt.

Die zu reinigenden Gegenstände werden aus einem Container 16 mit Pneumatik über die Zuleitung 17 in den Behandlungsbehälter 1 gefüllt.

Die Behandlung erfolgt in an sich bekannter Weise mit Wasser, Dampf, Heißluft und gegebenenfalls mit Detergenzien und/oder einer Silikondispersion. Da die Behandlung einschließlich der besonderen Führung der Behandlungsmedien im Behandlungsbehälter 1 zum Zwecke einer gründlichen und schonenden Behandlung der Gegenstände bekannt ist, wird im folgenden darauf nur kurz eingegangen.

In Figur 1 ist die Reinigungs- und Sterilisiermaschine unmittelbar nach dem Einsetzen des mit zu behandelnden Gegenständen gefüllten Behandlungsbehälters 1 in die Tragevorrichtung 4 der horizontalen Welle 3 dargestellt. Die Zu- und Abfuhrleitungen 13,14 sind über die jeweiligen Kupplungen 11,12 an den Behandlungsbehälter 1 angeschlossen. Die bis dahin geschlossenen Ventile 7,9 werden für die Behandlung geöffnet. Über die Leitungen 13,14 wird abwechselnd Heißwasser und Heißdampf eingeleitet, wodurch die Gegenstände im Dampfwirbelbett gereinigt werden.

Nach der Reinigung mit Heißwasser und Heißdampf werden die Gegenstände bei etwa 100 °C im Wirbelbett mit einer Silikondispersion silikonisiert. Nach beendeter Silikonisierung wird die Silikondispersion mit über die Leitung 13 zugeführter Druckluft nach unten gedrückt und über die untere Leitung 14 abgeleitet.

Nach der Silikonisierung wird dem Behandlungsbehälter 1 über die obere Leitung 13 zunächst Reinstdampf zum Sterilisieren der Gegenstände zugeführt, der über die untere Leitung 14 abgezogen wird, so daß der Dampfstrom alle Gegenstände erfaßt.

Anschließend werden die Gegenstände getrocknet, indem man über die obere Leitung 13 steril gefilterte und erhitzte Druckluft in den Behandlungsbehälter 1 einleitet, und diese über die untere Leitung 14 durch Vakuum abgesaugt, so daß der Luftstrom alle Gegenstände erfaßt.

Um sicherzustellen, daß die in dem Behandlungsbehälter 1 befindlichen Gegenstände gleichmäßig von den Behandlungsmedien erfaßt werden, ist es vorteilhaft, den Behandlungsbehälter 1 während oder zwischen den einzelnen Behandlungsphasen durch Drehen der horizontalen Welle 3 langsam hin und her zu bewegen. Durch die Bewegung erfolgt eine Durchmischung der Gegenstände.

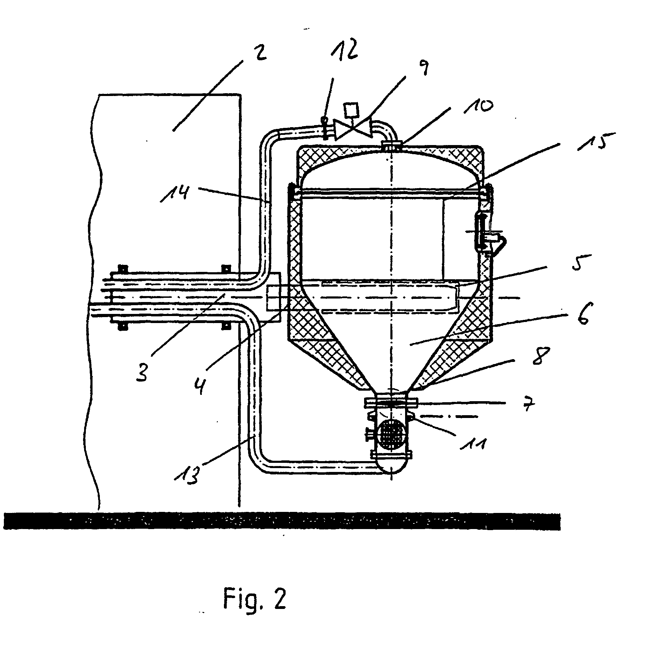

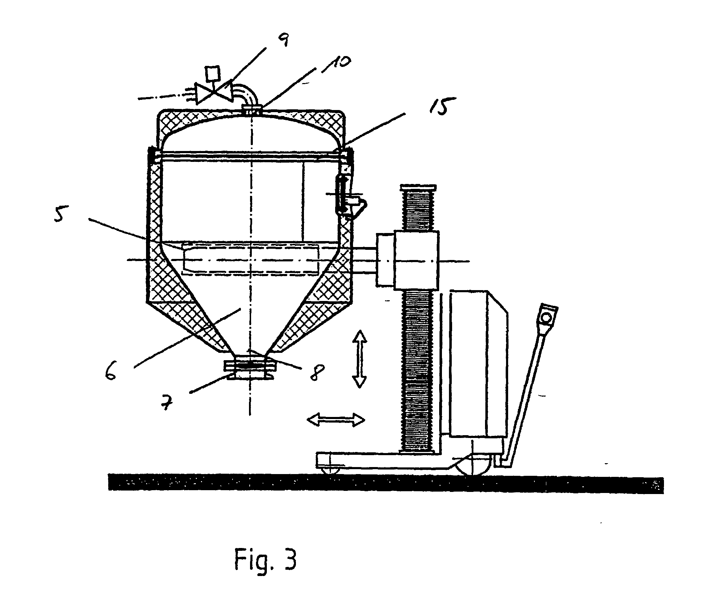

Nach erfolgter Behandlung werden der Behandlungsbehälteᵣ 1 wie in Figur 2 dargestellt um 180° gedreht, die Ventile 7,9 geschlossen, die Leitungen 13,14 abgekuppelt und der Behandlungsbehälter 1 mit Hilfe eines in Figur 3 dargestellten Transportfahrzeuges von der an der Welle 3 befestigten Gabel 4 abgezogen. Die gereinigten und sterilisierten Gegenstände befinden sich dann hermetisch verschlossen in dem Behandlungsbehälter 1, der sich seinerseits in nichtsterilisierter Umgebung befindet. Dieser kann dann zur Verbrauchsstelle der Gegenstände, etwa zu einer Arzneimittelabfüllanlage, transportiert werden.

## Patentansprüche

1. Reinigungs- und Sterilisiermaschine für kleine Gegenstände wie Verschlußelemente von pharmazeutischen Gefäßen, bestehend aus einem von einer horizontalen Welle (3) in einem Träger (2) getragenen und um die Achse der Welle (3) schwenkbar gelagerten Behandlungsbehälter (1), der ein trichterförmiges Oberteil (6) mit einer darin angeordneten, durch ein Ventil (7) verschließbaren Beund Entladeöffnung (8) aufweist und an dem von der Welle (3) getragene Zu- und Abfuhrleitungen (13,14) für mindestens ein Behandlungsmedium wie Wasser, Dampf, Druckluft angeschlossen sind, **dadurch gekennzeichnet, daß** die Welle (3) eine Tragevorrichtung aufweist, in der der Behandlungsbehälter (1) lösbar gehalten ist, und die Zu-und Abfuhrleitungen (13,14) über Kupplungen (11,12) lösbar mit an dem Behandlungsbehälter (1) angeordneten Ventilen (7,9) verbunden sind.

2. Reinigungs- und Sterilisiermaschine nach Anspruch 1, **dadurch gekennzeichnet, daß** die Welle (3) als Tragevorrichtung eine Gabel (4) aufweist, in die der Behandlungsbehälter (1) einhängbar ist.

3. Reinigungs- und Sterilisiermaschine nach Anspruch 1, **dadurch gekennzeichnet, daß** der Behandlungsbehälter seitliche Aufnahmen (5) aufweist, in die die Äste der Gabel (4) einschiebbar sind.

4. Reinigungs- und Sterilisiermaschine nach Anspruch 1, **dadurch gekennzeichnet, daß** das Ventil (7) der Be- und Entladeöffnung (8) an der Trichtermündung des Oberteils (6) angeordnet ist.

## Claims

1. A cleaning and sterilizing machine for small articles such as closure elements for pharmaceutical containers, said machine consisting of a treatment tank (1) which is supported by a horizontal shaft (3) in a carrier (2) and is mounted so that it can pivot about the axis of the shaft (3) and which has a funnel-shaped upper part (6) with a loading and unloading opening (8) arranged in it such that it can be closed by means of a valve (7), and inlet and outlet lines (13, 14) for at least one treatment medium such as water, steam or compressed air are connected to it and are supported by the shaft (3), **characterized in that** the shaft (3) has a supporting device in which the treatment tank (1) is held detachably, and the inlet and outlet lines (13, 14) are detachably connected by couplings (11, 12) to valves (7, 9) which are arranged on the treatment tank (1).

2. A cleaning and sterilizing machine according to claim 1, **characterized in that** the shaft (3) has a fork (4) as the supporting device in which the treatment tank (1) can be suspended.

3. A cleaning and sterilizing machine according to claim 1, **characterized in that** the treatment tank has lateral receptacles (5) into which the prongs of the fork (4) can be inserted.

4. A cleaning and sterilizing machine according to claim 1, **characterized in that** the valve (7) of the loading and unloading opening (8) is arranged at the mouth of the funnel of the upper part (6).

## Revendications

1. Appareil de nettoyage et de stérilisation pour des petits objets tels que des éléments d'obturation de récipients pharmaceutiques, constitué d'un récipient de traitement (1) supporté par un essieu (3) horizontal logé dans un support (2) et logé de manière à pouvoir être basculé autour de l'axe de l'essieu (3), récipient (1) qui comporte une partie supérieure (6) en forme d'entonnoir avec une ouverture de chargement et de déchargement (8) disposée dans cette partie supérieure et pouvant être fermée par une vanne (7), et auquel récipient (1) sont raccordées des conduites d'entrée et de sortie (13, 14) pour au moins un milieu de traitement, tel que de l'eau, de la vapeur, et de l'air comprimé, **caractérisé en ce que** l'essieu (3) comporte un dispositif de support, dans lequel le récipient de traitement (1) est maintenu de manière libérable, et **en ce que** les conduites d'entrée et de sortie (13, 14) sont reliées de manière libérable par l'intermédiaire de dispositifs d'accouplement (11, 12) à des vannes (7, 9) disposées sur le récipient de traitement (1).

2. Appareil de nettoyage et de stérilisation selon la revendication 1, **caractérisé en ce que** l'essieu (3) comporte, en tant que dispositif de maintien, une fourche (4), dans laquelle le récipient de traitement ((1) peut être suspendu.

3. Appareil de nettoyage et de stérilisation selon la revendication 1, **caractérisé en ce que** le récipient (1) comporte des logements latéraux (5), dans lesquels les branches de la fourche (4) peuvent être introduites par coulissement.

4. Appareil de nettoyage et de stérilisation selon la revendication 1, **caractérisé en ce que** la vanne (7) de l'ouverture de chargement et de déchargement (8) est disposée à l'embouchure d'entonnoir de la partie supérieure (6).
